# EUROPEAN PATENT APPLICATION

(11) **EP 1 685 857 A1**
(43) Date of publication of application: **02.08.2006**
(21) Application number: 05257740.0
(22) Date of filing: 15.12.2005
(51) Int. Cl.: A61L 15/58, C09J 153/00

(54) **An aborbent article having a skin contactable hot melt pressure sensitive adhesive**

(30) Priority: 16.12.2004 US 14128
(71) Applicant: JOHNSON & JOHNSON CONSUMER COMPANIES, INC., Skillman, NJ 08558 (US)
(72) Inventor: Luizzi, Joseph M., Newtown, PA 18940 (US)
(74) Representative: Mercer, Christopher Paul

(57) **Abstract**

There is provided an absorbent article having a skin contactable hot melt pressure sensitive adhesive composition, which provides improved adhesion to skin. The pressure sensitive adhesive uses an endblock softening agent. The endblock softening agent contained in the adhesive's composition is preferably a low softening point aromatic resin tackifier.

## Description

### FIELD OF THE INVENTION

The present invention relates to a hot melt pressure sensitive adhesive composition for attachment means and to absorbent articles having a layer of the hot melt pressure sensitive adhesive composition, which provides improved adhesion to skin. The articles of the present invention are particularly useful for medical tapes, bandages and wound strips.

### BACKGROUND OF THE INVENTION

In the area of prior art for the application of adhesives directly to human skin, solvent, water-based, and hot melt adhesives are known. It is well known by those skilled in the art that skin adhesives for applications such as wound care, based on acrylic polymers are typically used commercially, because they provide consumers with superior wear and durability performance versus adhesives based on thermoplastic elastomers. Although acrylic pressure sensitive adhesives have long been noted to those skilled in the art as effective for medical tapes, bandages, wound strips, etc., they are costly and require drying ovens to evaporate the carrier solvent or water. In addition, solvent based systems present issues with regard to safety and environmental concern. Hot melt adhesives have been used as well, however from a performance standpoint, they have not been as effective in wear times and durability, both of which relate to the absorbent article, the adhesive used and its' composition.

Hot melt adhesives based on olefins such as low-density polyethylene and ethylene copolymers like ethylene-vinyl acetate are well known. Other polymers based on acrylic chemistries are also known. Many inventions are based on block copolymer type hot melt adhesives, such as styrene-butadiene-styrene ("SBS") block copolymer. The inventions either emphasize different ratios of di-block vs. triblock, or on various applications.

The use of hot melt adhesives based on block copolymers of linear or radial co-polymer structures having the formula (A-B)_{X} wherein block A is a polyvinylarene block, block B is a poly(monoalkenyl) block, x denotes the number of polymeric arms, and wherein x is an integer greater than or equal to one are well established in the art. For example, U.S. Patent No. 3,239,478 to Harlan teaches the art of making arylene-diene block copolymer based hot melt adhesives, specifically using blends of SBS or styrene-isoprene-styrene ("SIS"), mid-block tackifying resing and mid-block plasticizers. Noting their application in areas such as pressure sensitive tapes, such as masking tapes, adhesive sheets, primers for other adhesives, adhesive tapes, mending tapes, electrical insulation tape, laminates, hot-melt adhesives, mastics, cements, caulking compounds, binders, sealants, pressure sensitive adhesives otherwise, delayed tack adhesives, shoe sole adhesives, cloth backings, carpet backings, and cements.

U.S. Patent No. 4,080,348 and 4,136,071 to Korpman discloses hot melt adhesives based on linear poly(Styrene-lsoprene-Styrene) (SIS) or radial p(SI)n block-copolymer thermoplastic elastomer and p(SI) diblock copolymer. This adhesive is noted as possessing superior "finger tack" and skin adhesion, as well as the ability to adhere to oily surfaces.

U.S. Patent No. 5,891,957 to Korpman discloses a hot melt adhesive composition for skin adhesion and bandage applications based on poly(Styrene-Isoprene-Styrene) (SIS) block-copolymer thermoplastic elastomer. The adhesive composition of this invention includes about 20-300 parts, hydrocarbon resin component per one hundred parts by weight of the thermoplastic elastomeric component. The resin component consists essentially of tackifier resins for the elastomeric component.

The use of end-block plasticizing resins for thermoplastic elastomer based adhesives is known in the art. To those skilled in the art it is noted for reducing process viscosities, as well as enhancing wetting and adhesion characteristics, or in the case of reinforcing resins and solidifying plasticizers, reducing tack or increasing thermal performance.

U.S. Patent No. 6,391,960 to Sambasivam et al. discloses a hot melt adhesive comprising end block resin in combination with a radial or linear styrene-butadiene-styrene ("SBS") block copolymer having a solution viscosity greater than 1000 cPs at 25% in toluene suitable for use in construction and elastic attachment adhesion. This adhesive is noted for its' application non-woven assembly as well as for the end block's reinforcing resin. However, Sambasivam does not incorporate or suggest the use of an absorbent article using an end block modifier within the adhesive's composition for improved adhesion to the skin. As these formulations are predominated by high loadings of mid-block tackifying resins, and oils, their use in adhesion to skin would results in significant cold flow, cohesive failure and the leaving of residue on the skin after removal as well as being too aggressive on the skin. The reinforcing resin stiffens the end block, rather than plasticizing it thereby providing softening attributes.

U.S. Patent No. 5,627,229 to Bunnelle et al. discloses a hot melt adhesive comprising a cyclohexane dimethanol dibenzoate plasticizer, tackifier and a thermoplastic polymer. Although the adhesive composition is taught for use in a variety of construction applications, this aromatic plasticizer undergoes a secondary crystallization slowly after application, resulting in a significant reduction in tack and pressure sensitive character. Thus it is not suitable as a pressure sensitive, more specifically for adhesion to skin.

U.S. Patent Application Publication 2003/010513 discloses a hot melt adhesive comprising an effective amount of a scented material having a closed cup flash point of greater than 100 degrees F. Although the hot melt adhesive is taught for used in for wound care application, the hot melt adhesive does not contain end block modifiers for improved adhesion to skin.

It would be desirable to have an absorbent article having a skin contactable hot melt pressure sensitive adhesive that has improved adhesion to the skin, and longer wear times. It would further be desirable to improved wet adhesion to skin. Surprisingly, it has been found that the use of end block softening plasticizers and resins in S-I-S based adhesives provides superior wetting and adhesion to the skin surface, resulting in improved adhesion to skin, longer wear times and durability. Thus providing performance characteristic comparable to acrylics, with the cost and manufacturing advantages of hot melt adhesives

### SUMMARY OF THE INVENTION

In accordance with this invention, there is provided an absorbent article having a skin contactable hot melt adhesive, comprising:
a.) an backing material having a skin contacting surface and a second surface; b.) an absorbent pad on a portion of the skin contacting surface of the backing material; and c.) a hot melt adhesive comprising a block copolymer, mid-block tackifying resin, mid-block plasticizer, an end-block softening agent, wherein the hot melt adhesive is on the skin contacting surface of the backing material.

Another aspect of the present invention is directed to a pressure sensitive hot melt adhesive composition for absorbent articles having improved adhesion to skin.

The invention will be more fully understood and further advantages will become apparent when reference is made to the following detailed description of the invention.

### DETAILED DESCRIPTION OF PREFERRED EMBODIMENTS

The invention discloses an absorbent article having a backing material, a hotmelt adhesive, and a absorbent pad for contacting the skin. As used herein, the term "absorbent" shall mean any material or composite that absorbs fluid, especially wound exudates.

The backing materials of the present invention may be any thin, flexible materials that are capable of conforming to a user's body in use and include, but are not limited to, flexible polymeric films, including polyolefin films such as polyethylene and polypropylene films; polyvinylchloride films; and ethylene-vinyl acetate films. Other useful backing materials include nonwoven fabrics, woven fabrics, and laminates of polymeric films with woven fabrics or nonwoven fabrics. A woven backing material particularly useful for practice of the invention has polyester yarns such as polyethylene terephthalate or polybutylene terephthalate yarns in the warp direction and polyamide yarns, such as nylon 6 or nylon 6,6 yarns, in the fill direction. Alternatively, the woven backing material may have polyethylene terephthalate yarns in the warp direction and polybutylene terephthalate yarns in the fill direction. Such woven backings are known and are commercially available.

In a preferred embodiment, the absorbent article is breathable. Breathable absorbent articles may be formed by utilizing breathable backing materials such as non-woven fabrics, woven fabrics, micro porous films, and the like. If the backing material is not inherently breathable, then the desired breathability may be obtained by perforating the backing material as is known in the art. Backing materials for use in the practice of the present invention are preferably breathable.

Apertured films are useful as backing materials in the practice of the invention. Such apertured films are breathable films. Particularly useful apertured films include Vispore® Brand apertured film supplied by Tredegar under the designations Tredegar X-6799, Tredegar X-6845, Tredegar X-6923, Tredegar X-6944, and Tredegar X-6844. Apertured films may be made from any polymeric material including, but not limited to polyethylene, metallocene catalyzed polyethylene, polypropylene, polyolefin copolymers, and ethylene vinyl acetate copolymers. The preferred backing of the present invention is a polyolefin film from Tredegar, X-27939.

The absorbent article of the present invention also contains a pressure sensitive hot melt adhesive coated onto a surface of the backing material that is intended to contact the skin of a wearer in use. Suitable hot melt adhesives for use in the invention are formed from block copolymers including linear or radial co-polymer structures having the formula (A-B)_{X} wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to styrene-isoprene-styrene, styrene-butadiene-styrene, styrene-ethylene-butadiene-styrene, styrene-ethylene-propylene-styrene, conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton™ elastomers from Shell Chemical Company, Vector™ elastomers from Dexco, Solprene™ from Enichem Elastomers and Stereon™ from Firestone Tire & Rubber Co. The block copolymers preferably have a melt flow index ranging between about 1 and about 50.

Suitable mid-block tackifying resins include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic/aromatic petroleum resins and the hydrogenated derivatives thereof, and combinations thereof. Commercial examples of these types of resins include Foral® hydrogenated rosin ester, Staybelite® hydrogenated modified rosin, Poly-pale® polymerized rosin, Permalyn® rosin ester, Pentalyn® rosin ester, Adtac® oil extended hydrocarbon resin, Piccopale® aromatic hydrocarbon, Piccotac®, Hercotac® aromatic modified aliphatic hydrocarbon, Regalrez® cycloaliphatic resins, or Piccolyte® from Hercules, Escorez® from Exxon Chemical aliphatic hydrocarbon and cycloaliphatic resins, Wingtack® from Goodyear Tire & Rubber Co. synthetic polyterpene resins including aromatic modified versions, Arkon® partially and fully hydrogenated aromatic resins from Arakawa Chemicals, Zonatac® styrenated terpene resin, Zonarez® rosin ester and Zonester® rosin ester from Arizona Chemical and Nevtac® aromatic modified aliphatic hydrocarbon from Neville Chemical Company. The mid-block tackifying resins have a softening point temperature of at least 75°C.

Suitable mid-block plasticizers in include synthetic liquid polyterpene resins like Wingtack 10, from Goodyear Chemcial, and aliphatic white mineral oil, such as Kaydol from Crompton. The mid-block plasticizers have a softening point temperature of less than 25°C.

The adhesive further comprises end block softening agents. The end block softening agents are aromatic hydrocarbon resins having a low molecular weight from about 500 to about 1200. The resins soften and tackify the end blocks of the block copolymer. Suitable end block softening agents, consisting of either resins or plasticizers, include benzyl butyl phthalate, di-octyl phthalate, and liquid hydrocarbon resins based on aromatic petroleum feeds or pure styrene or alphamethyl styrene feeds. Examples of theses include Piccovar and Piccolastic resins from Eastman Chemical and Nevchem NP resins from Neville Chemical. The end block softening agent preferably is NP-25, more preferably NP-10. In a preferred embodiment the NP-10 is from about 10 % w/w to about 15 % w/w of the total composition for the hot melt adhesive.

The absorbent article of the present invention also includes an absorbent pad. The absorbent pad may be made from various materials including rayon fibers; natural fibers, such as, but not limited to, cotton and wood pulp fibers, and synthetic fibers, such as, but not limited to, polyester, polyamide, and polyolefin fibers. Synthetic fibers comprising two or more polymers may be used. Blends of fibers may be used. The fibers may be bi-component fibers. For example, the fibers may have a core of one polymer, and a sheath of a different polymer. The denier of the fibers comprising the absorbent pad is not limited, but typically ranges from about 3 to 10 denier.

The basis weight of the absorbent pads is not limited, but typically ranges from 0.003 g/cm² to 0.015 g/cm². The size of the absorbent pad may vary depending on the size of the bandage and/or the size of the wound to be protected or treated.

### EXAMPLES

### Example 1

The following examples are merely illustrative and not intended to limit the scope of the present invention in any manner.

The adhesive can be applied to the absorbent article in any desired manner, e.g., by spraying, screen printing or slot die coating. The amount of adhesive typically applied is well known in the art however generally, the adhesive coating weight may vary from about 20 grams per square meter ("gsm") to about 100 gsm.

In a preferred embodiment, the hot melt adhesives of the present invention comprise (by weight):
about 20 - 60% block copolymer;
about 20 - 80% mid block tackifying resin;
about 0 - 40% mid-block plasticizer;
about 5 - 30% end-block softening resin or plasticizer
and about 0 - 2.0% antioxidant.

The hot melt adhesive of the present invention was prepared using the following procedure:
In a 600cc Brabender mixer fitted with sigma blades (C.W. Brabender Instruments, Inc., South Hackensack, NJ), heated to about 162° C, 125.57 grams of Kraton D-1113X (Kraton Polymers), 285.17 grams of Wingtack Plus, 156.41 grams of Wingtack 10 (Goodyear Tire & Rubber Co.) and 3 grams of Ethanox 330 (Ethyl Corp.) were added and melt mixed until homogenous. Once homogenous, 30 grams of-benzyl butyl phthalate was added. The contents were then mixed for 10 minutes at 60 rpm. The resulting adhesives were hot melt coated at a weight of 50 gsm coat weight onto bandage backings. The preferred backing was a 0.004" thick polyolefin film from Tredegar, X-27939. The adhesives were coated onto the backings using a slot coating head. Each bandage strip was die cut, and gauze pads were affixed to the adhesive.

### Example 2

In accordance with this example , aromatic plasticizing resins were used. The following table charts 24 hour wear test results of various loadings of NP-25, NP-10 (Neville Chemical, Co.) and with no additive as a control. However since the control was tested using an old protocol in which the adhesion scale is from 1 to 7, it was normalized to a 1-5 scale in order to compare with the rest of the data. Further, "hours on fingers" and "hours on arms" may also be indicative of skin adhesion strength.

**Table 1**

| | No additive | 10% aromatic hydrocarbon resins (NP-25) | 15% aromatic hydrocarbon resins (NP-25) | 20% aromatic hydrocarbon resins (NP-25) | 25% aromatic hydrocarbon resins (NP-25) | 30% aromatic hydrocarbon resins (NP-25) | 10% aromatic hydrocarbon resins (NP-10) | 15% aromatic hydrocarbon resins (NP-10) |
|---|---|---|---|---|---|---|---|---|
| | | | | | | | | |
| Hours on Fingers (Max 24) | 19.1 | 20.314 | 21.15 | 21.513 | 21.135 | 22.276 | 22.25 | 22.34 |
| Adhesion to finger (1-5) | 2.79* | 3.116 | 4.24 | 3.667 | 3.667 | 4 | 3.821 | 3.879 |
| | | | | | | | | |
| Hours on Arms (Max24) | 22.1 | 21.971 | 23.25 | 23.519 | 23.474 | 23.641 | 23.628 | 23.712 |
| Adhesion to Arms (1-5) | 3.39** | 3.651 | 5.69 | 4.385 | 4.231 | 4.462 | 4.436 | 4.436 |

| | | | | | | | | |
|---|---|---|---|---|---|---|---|---|
| * 3.9x5/7=2.79 (normalized from 1-7 scale to 1-5 scale) | | | | | | | | |
| ** 23.2x5/7=3.39 (normalized from 1-7 scale to 1-5 scale) | | | | | | | | |
| NP-10: Melting Point of 10°C | | | | | | | | |
| NP-25 : Melting Point of 25°C | | | | | | | | |

Table 1 demonstrates that the percentage of end block softening resins within the composition is critical, in that if it's too low the adhesive is less able to wet and adhere to skin. Conversely, if the softening resin percentage amount is too high the adhesive composition would flow off the backing material at room temperature thereby causing debris to adhere to the edges of a backing material. The softening point of the end-block resin relates to the resin's molecular weight in that, these resins are amorphous materials. Thus when heated the polymer chain changes from a solid to a flowable viscous material, further if the softening point temperature is too low (below 0°C) then the adhesive will flow off the backing material at room temperature and conversely if too high (above 65°C) then the adhesive would not have sticky adhesion.
The specification above is presented to aid in the complete and non-limiting understanding of the invention disclosed herein. While the primary use for the adhesive composition of the present invention is in wound care, the compositions of the invention can also be used in entirely different applications, where adhesion to the skin is desired.

Since many variations and embodiments of the invention can be made without departing from its spirit and scope, the invention resides in the claims hereinafter appended.

## Claims

1. An absorbent article having a skin contactable hot melt adhesive, comprising:
a.) a backing layer having a first surface adapted to contact a user's skin in use and a second surface opposite the first surface; b.) an absorbent pad; and c.) a hot melt adhesive comprising a block copolymer, mid-block tackifying resin, mid-block plasticizer, an end-block softening agent and an antioxidant, wherein the hot melt adhesive and the absorbent pad are on the first surface.

2. The absorbent article of claim 1 wherein the absorbent article is a disposable bandage.

3. The absorbent article of claim 1 wherein the hot melt adhesive comprises poly aromatic alkenyl block copolymer, hydrocarbon tackifying resin, aliphatic plasticizer, antioxidants and an aromatic endblock plasticizing agent.

4. The absorbent article of claim 3 wherein said endblock plasticizing agent is a low molecular weight aromatic hydrocarbon resin derived from petrochemical feedstocks.

5. The absorbent article of claim 1 wherein said block copolymer is a styrene-isoprene-styrene copolymer having a melt flow index ranging between about 1 and about 50.

6. The absorbent article of claim 1 wherein said mid-block tackifying resin is selected from a group consisting of an alphatic and an aromatic modified aliphatic resin having a softening point temperature of at least 75°C.

7. The absorbent article of claim 1 wherein said mid-block plasticizer is selected from a group consisting of a mineral oil, paraffin oil, napthenic oil, aliphatic resin and aromatic modified aliphatic resin having a softening point temperature less than 25°C.

8. The absorbent article of claim 4 wherein said end-block plasticizing agent has a molecular weight of less than 750 gsm.

9. A hot melt adhesive comprising:
(a) about 20 to 60 weight percent of a styrene-isoprene-styrene block copolymer;
(b) about 20 to 80 weight percent of mid-block tackifying resin;
(c) about 0 to 40 weight percent of mid-block plasticizer;
(d) about 5 to 30 weight percent of end-block softening agent; and
(e) about 0 to 2.0 weight percent antioxidant.

10. The hot melt adhesive of claim 9 wherein the end-block softening agent has a softening point of less than 30°C.

11. The hot melt adhesive of claim 9 wherein the end-block agent is selected from the group consisting of benzyl butyl pthalate, di-octyl pthalate, and liquid aromatic hydrocarbon resins.

12. The hot melt adhesive of claim 9 wherein the end-block agent is an aromatic hydrocarbon resins having a melting point at 25°C.

13. The hot melt adhesive of claim 9 wherein the end-block agent is an aromatic hydrocarbon resins having a melting point at 10°C.

14. The hot melt adhesive of claim 13 wherein said aromatic hydrocarbon resin is from about 10% w/w to about 15% w/w of the total composition for said hot melt adhesive.
